# EUROPEAN PATENT SPECIFICATION

(11) **EP 4 268 732 B1**
(45) Date of publication and mention of the grant of the patent: **08.07.2026**
(21) Application number: 22195104.9
(22) Date of filing: 12.09.2022
(51) Int. Cl.: A61B 17/04, A61B 1/32, A61B 17/06, A61B 1/303, A61B 17/00

(54) **INSTRUMENTS FOR SUPPORTING AND PLACING RESORBABLE THREADS IN THE SUB URETHRAL SPACE FOR THE NON-SURGICAL TREATMENT OF GRADE 3 STRESS URINARY INCONTINENCE IN WOMEN**
INSTRUMENTE ZUR UNTERSTÜTZUNG UND PLATZIERUNG RESORBIERBARER FÄDEN IN DER UNTERURETHRALEN KAMMER ZUR NICHT-CHIRURGISCHEN BEHANDLUNG VON HARNINKONTINENZ DER STUFE 3 BEI FRAUEN
INSTRUMENTS DE SUPPORT ET DE MISE EN PLACE DE FILS RESORBABLES DANS L'ESPACE SOUS-URETRAL, POUR LE TRAITEMENT NON CHIRURGICAL DE L'INCONTINENCE URINAIRE DE NIVEAU 3 CHEZ LA FEMME

(30) Priority: 27.04.2022 UY 39739
(43) Date of publication of application: 01.11.2023
(73) Proprietor: Luksenburg Wiernik, Ariel, Montevideo (UY)
(72) Inventor: Luksenburg Wiernik, Ariel, Montevideo (UY)
(74) Representative: Pons IP

(56) References cited:
- WO-A1-2009/064866
- WO-A2-2008/067317
- US-A1- 2002 005 204
- US-A1- 2008 139 877
- US-A1- 2008 210 248
- US-A1- 2009 306 459

## Description

### A).- INTRODUCTION

The invention refers to instruments for supporting and placing polydioxanone or polycaprolactone resorbable threads in the non-surgical and minimally invasive treatment of grade 3 stress urinary incontinence in women.

Independent claim 1 defines the invention, with further aspects provided in the dependent claims.

The patient tries to tension and restore support to the sub urethral spaces and vaginal urethra, an anatomical site through which the urethra passes and rests on in its path from the bladder to the urethral meatus. The weakening or flaccidity of these anatomical spaces caused by childbirth or hormonal deficits, among others, determines the so-called *"urethral hypermotility",* one of the main known causes responsible for true stress urinary incontinence.

Currently, the surgical technique employed in cases of true urine incontinence is the technique called *Trans Obturator Tape (TOT),* consisting of the application and placement of a mesh of non-resorbable material under the urethra, in this way rectifying its path. Such a mesh usually results in a reaction of the organism, which forms a fibrosis around it as a result of the organism's reaction to this foreign body. In the state of the art are known the following documents:
- US 2002/005204 A1, which discloses surgical treatment of stress urinary incontinence.
- US 2009/306459 A1, which discloses a Surgical procedure for the treatment of female urinary incontinece: tension-free inside-out transobturator urethral suspension.
- US 2008/139877 A1, which discloses systems, methods and devices relating to delivery of medical implants.
- US 2008/210248 A1, which discloses a system for the treatment of stress urinary incontinence.
- WO 2008/067317 A2 which discloses a method and apparatus for supporting body organs, urethra and bladder neck.
- WO 2009/064866 A1 which relates to implants having two arms and a support portion configured to support a body tissue, such as a urethra.

The surgical procedure described was the gold standard for many years. However, due to the complications and its relative effectiveness, after more than 20 years it has been discouraged by several countries with the result of many of them returning to the previous technique - the modified Burch operation - which involves a more complex surgical activity and with a longer postoperative period.

The novel technique whose authorship and patentability are claimed is aimed at those patients who have severe urinary incontinence, grade 3 (at minimum efforts), who until now had only a surgical solution.

The technique consists of placing a network of resorbable threads of different types in the sub urethral and vaginal urethral spaces (lateral to the urethra), forming a crossed network in the latter *(sub urethralspyder web),* and another on both sides of the urethra, in the vaginal urethral space. In this way, a mesh of absorbable threads is completed, from one ischiopubic ramus to the other, located throughout the vaginal urethral space.

This innovative technique has a double mechanism of action; one immediate and the other in the medium and long term. The urethra is immediately provided with a new physical support in its entirety (approximately 3 cm), with the placement of a network of resorbable threads in the sub urethral space. In the same way as the surgical technique, the threads will generate a foreign body reaction and trigger a fibrosis reaction around them, thereby enhancing - in the medium and long term - the support effect that is provided when the thread network is placed.

The great advantage of this technique is that it is an ambulatory in-office procedure, painless, without postoperative period and allowing immediate return to work.

For its execution, the use of a series of threads and instruments is foreseen, which facilitate and guide the procedure in a safe way and avoiding errors and iatrogenic injury, as well as the use of specific inputs.

### 1).- THREADS

### 1.1) .- POLYCAPROLACTONE (PCL) OR POLYDIOXANONE (PDO) THREADS:

PDO and PCL threads are very popular in the aesthetic field. They are resorbable and are mounted inside a needle or cannula with a blunt tip, protruding through the tip to move back in the opposite direction and attach externally to the needle or cannula, held in place by a polyurethane material.

For the execution of the described technique, the threads have special dimensions (according to the anatomy of the urethra and the vaginal urethral space), and their application consists of introducing them by mounting them on appropriate instruments -also novel- that will be described.

The application of such threads is similar for the different sizes and models. For their application they are introduced through the vaginal urethral space by being mounted on the aforementioned instruments, and once the needle or cannula accesses the desired anatomical site (sub urethral and lateral spaces to the urethra) they are removed, with the result that, when coming into contact with the tissues, the threads are placed in the same place where they were originally introduced.

### 1.2) .- MESH THREADS (9):

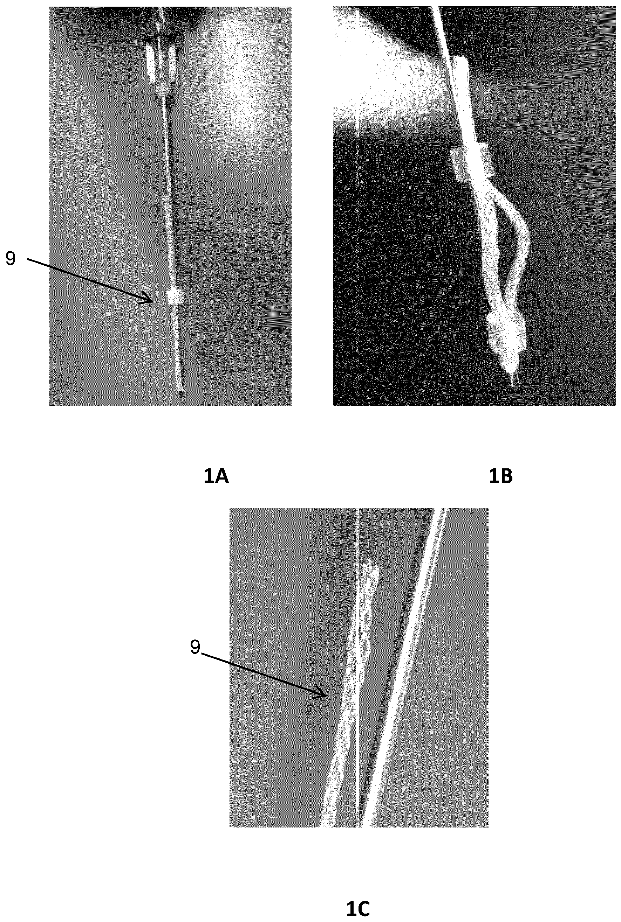

The threads are shown in figure 1A-1C. The mesh threads are made of six 6.0 threads, intertwined and inserted into a 19G 38 mm cannula, with blunt tip to avoid injury during the trajectory of use. For their application it is necessary to make an inlet orifice with a needle.

### 1.3) .- MULTIPLE THREADS:

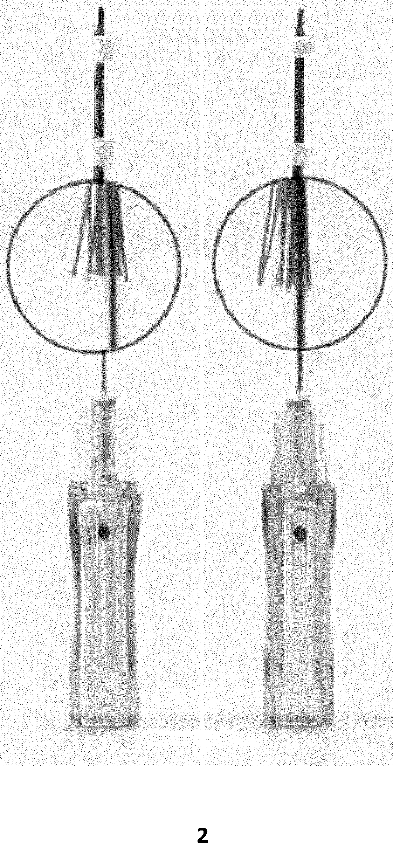

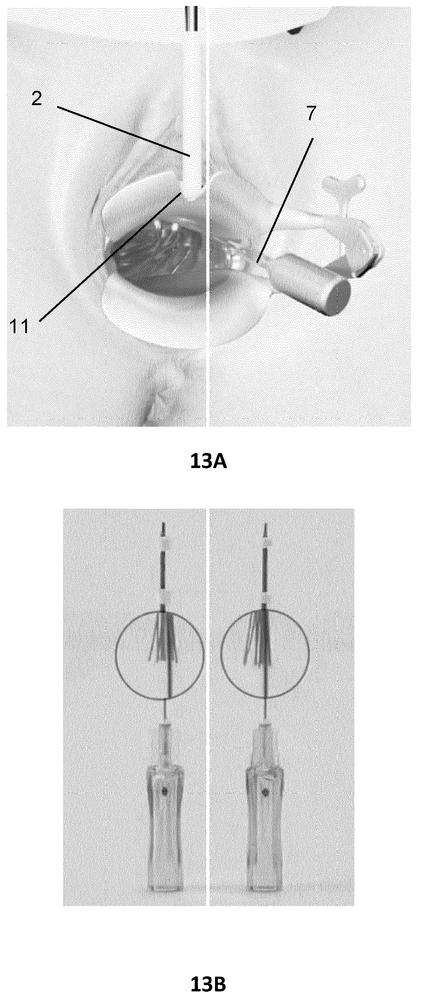

The multiple threads are shown in figure 2 and figure 13B. Multiple threads consist of five threads. Like the mesh threads, they are presented inside a 21G 38 mm blunt cannula ending at a type of "ponytail". As in the previous case, their use requires previously making an inlet orifice with a suitable needle.

### 1.4) .- MONOSCREW THREADS (10):

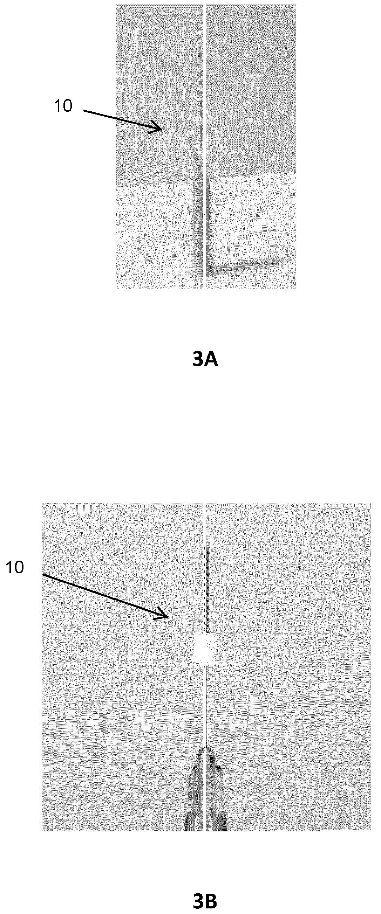

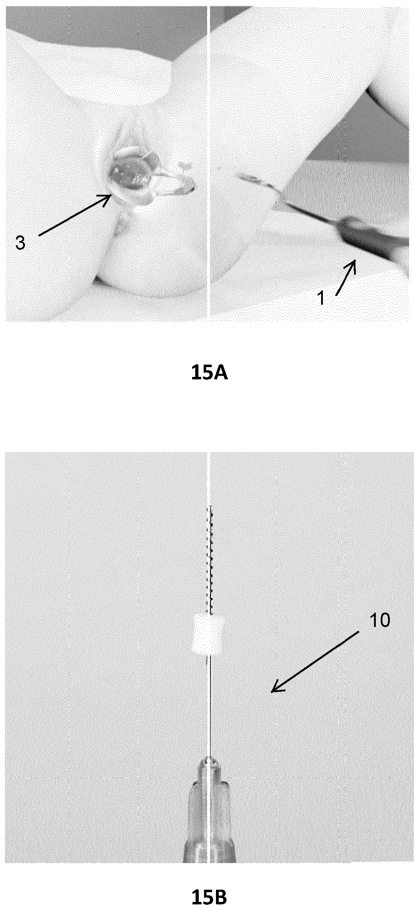

They are simple turned threads (MONOSCREW 26g 30 MM) that are mounted on a needle (8) with a cutting edge. The monoscrew (10) is shown in figure 3A and 3B and figure 15B.

### 2).- THE INSTRUMENTS:

### 2.1) .- FENESTRATED SPECULUM (3)

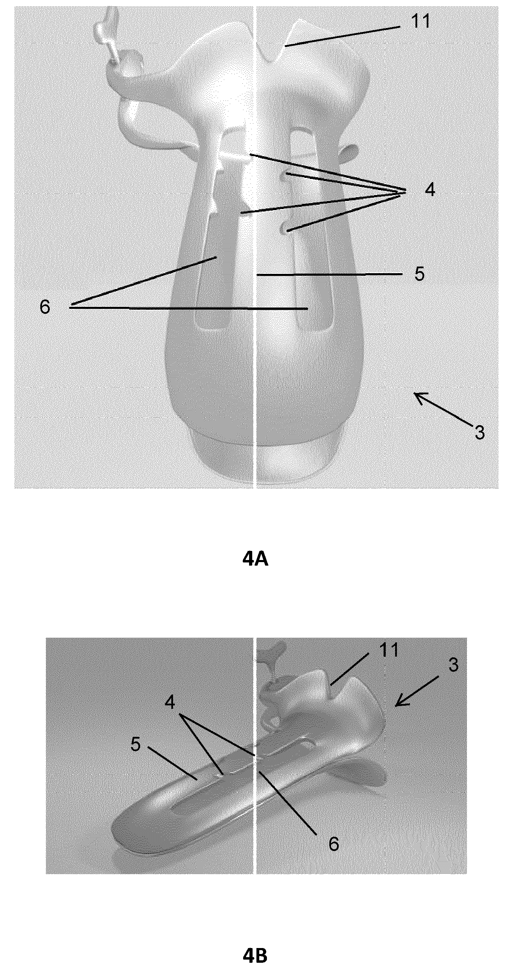

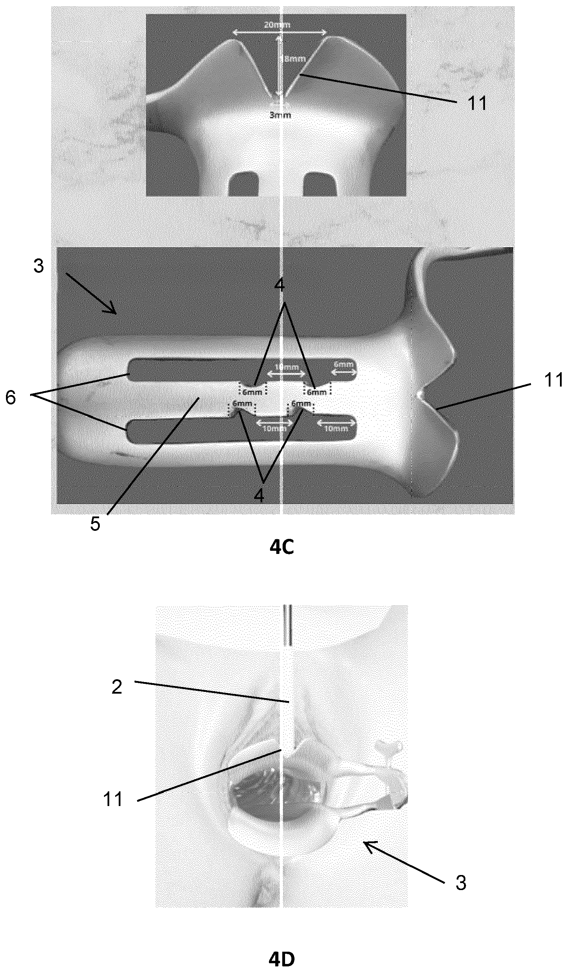

The fenestrated speculum (3) is shown in figure 4A-4D. It is made of stainless steel, it has two lateral and longitudinal windows (6), 7 mm wide by 65 mm long.

In its center and between both windows (6), it has a central and longitudinal area ("central rib" (5)), whose function is to provide stability to the speculum and mechanical protection to the urethra during the procedure. On the inner edges of each window (6) there are four inclined grooves (4) whose function is to guide the operator during the procedure, orienting and delimiting the area where the needles (8) must be inserted with the Monoscrew thread (10) mounted on needles (8). In turn, the inclined grooves (4) will guide the technician in the application of the multiple threads.

It also has a notch at 12 o'clock, which serves the supporting function when using the LS3 Introducer, thereby protecting the urethra when suburethral or near urethral threads are applied.

### 2.2) .- INTRODUCER (2):

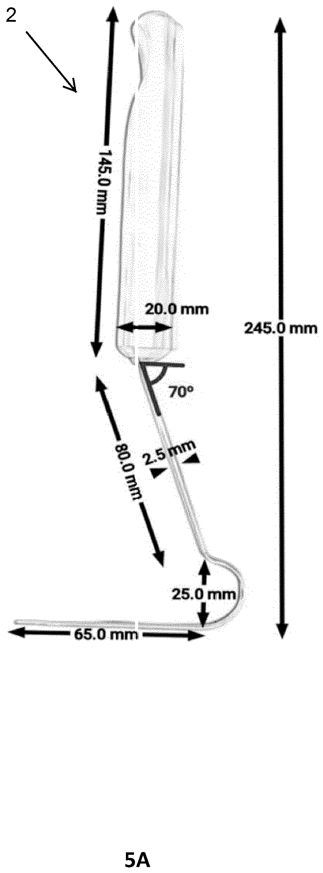

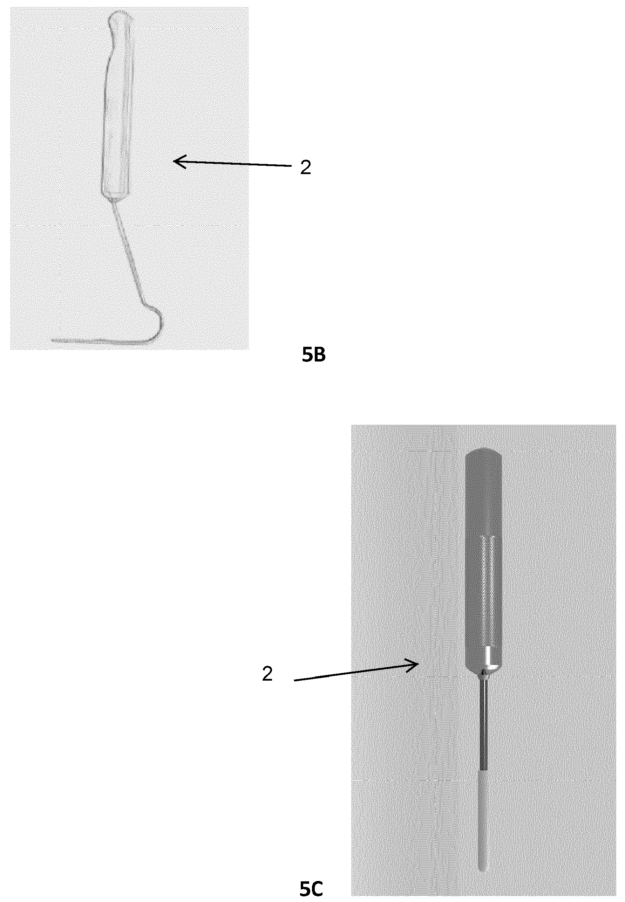

The introducer (2) is an instrument made of stainless steel, with the measurements and shape described in figure 5 A, which is always used covered from its distal end with a sterile bladder probe n° 18 (figure 5 C).

The introducer (2) comprises a handle or clamp followed by a straight portion and a hook at its end, shaped and sized for entry into the urethra through the urethral meatus and into the bladder. According to Fig.5A, there is an angle of 70° between the handle and the straight portion.

With its handle held and facing upwards, it enters the urethra through the urethral meatus (perpendicular to it), and is introduced (2) through the entire urethra to the bladder.

Once located, it rises towards the zenith and deploys the vulvar vestibule, elevating the urethra to open the sub urethral space, thereby providing safety and protection to the urethra during the placement of threads in the sub urethral space.

### 2.3) .- BAYONET (1):

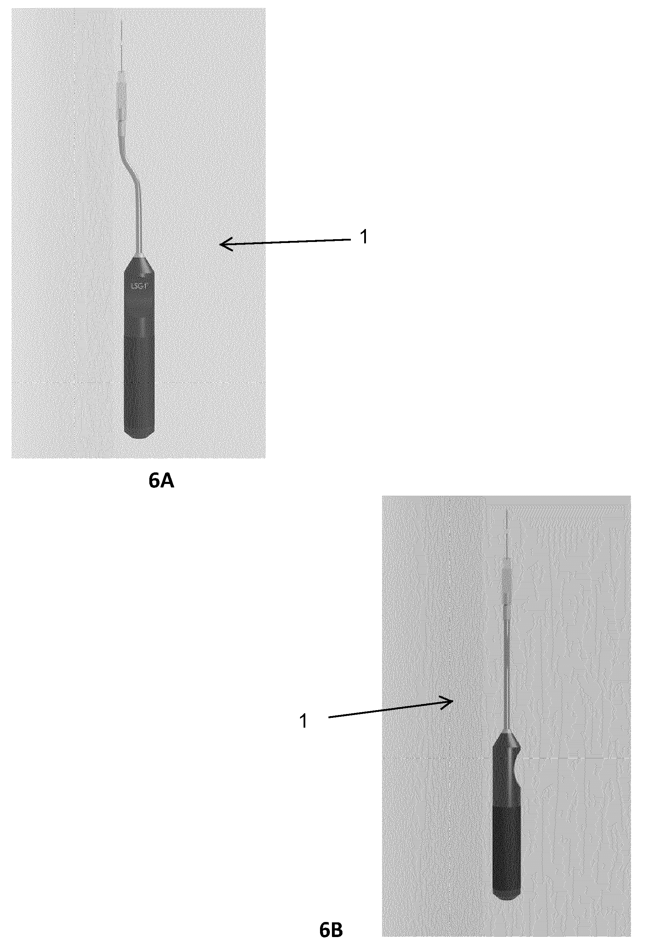

The bayonet (1) is shown in figure 6A-6B. It is an instrument developed for the intravaginal application of threads. It represents an "extension of the technician's hand" and allows easy maneuvering inside the vagina when placing the threads on its anterior face.

It measures 23.5 cm in all its extension and has a handle of 10 cm and a rod of 13.5 cm in length, in the shape of an italic 'S' and at an angle of 30°. It ends with a pressure-adaptive pointer to the back of the needle or cannula carrying the thread.

This instrument is used to place multi threads and monoscrew threads (10). For the former, once the cannula has been placed on the pointer of the bayonet (1), and following the path of the inclined groove (4) of the speculum windows (6), it is applied on the anterior face of the vagina with the fenestrated speculum (3) located at 12 o'clock. It enters at a 20° angle for 1 cm and then rectifies its path until completing its full introduction.

The same instrument is also used to place the monoscrew threads (10) with needles (8) in the vaginal urethral space, laterally to the urethra. Once the needle (8) with the monoscrew thread (10) has been placed on the pointer of the bayonet (1), the speculum is rotated 1 cm to the right and through the right window of the same, and 3 threads are applied along the longitudinal axis of the speculum.

The application of the threads on the anterior face of the vagina is achieved by entering at an angle of 20° for a distance of 1 cm, and then rectifying its path until its full introduction. Once this is done, the fenestrated speculum (3) is rotated twice more to the right, in the direction of the right ischiopubic ramus, and another three threads are placed in each rotation, repeating the same maneuver to the left.

### 2.4) .- THREAD HOLDER (7):

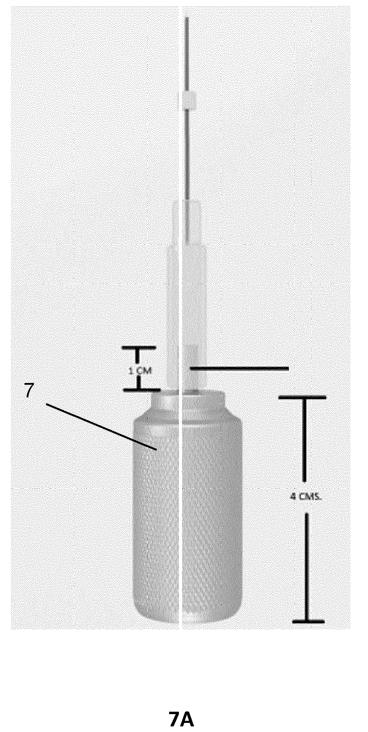

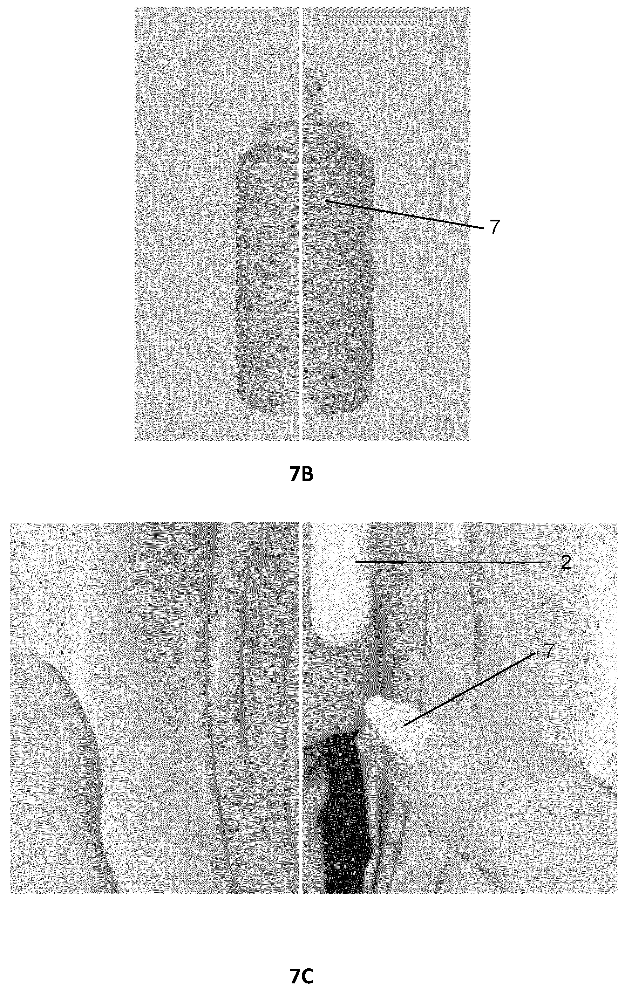

This solid stainless steel instrument has a cylindrical body 4 cm long and 1.8 cm in diameter, and a pressure-adaptable pointer where the mesh threads will be placed for placement (figures 7A, 7B and 7C).

### B).- DESCRIPTION OF THE TECHNIQUE:

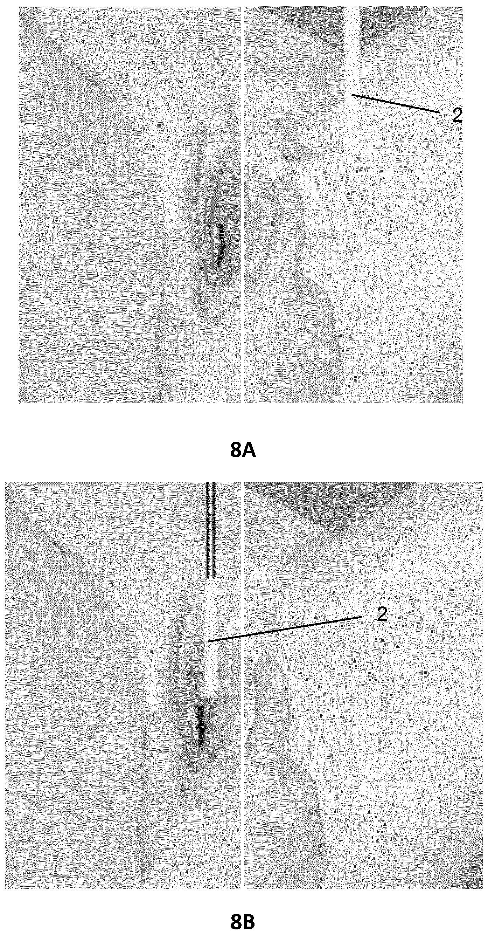

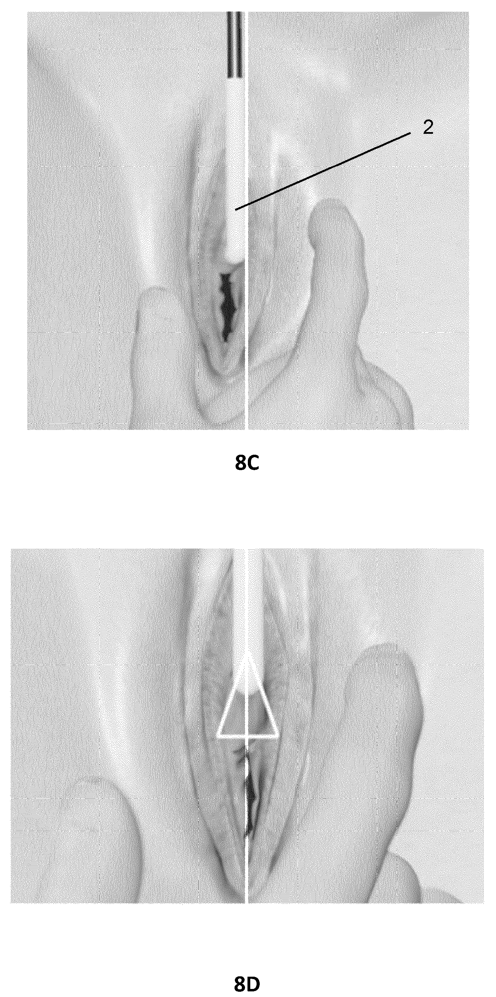

After applying topical anesthesia in the vulva and vagina, the introducer (2) is placed through the urethral meatus **(****figures 8A and 8B****)** and then raised to allow the elevation of the urethral meatus and the elongation of the vulvar vestibule **(****figure 8D****).**

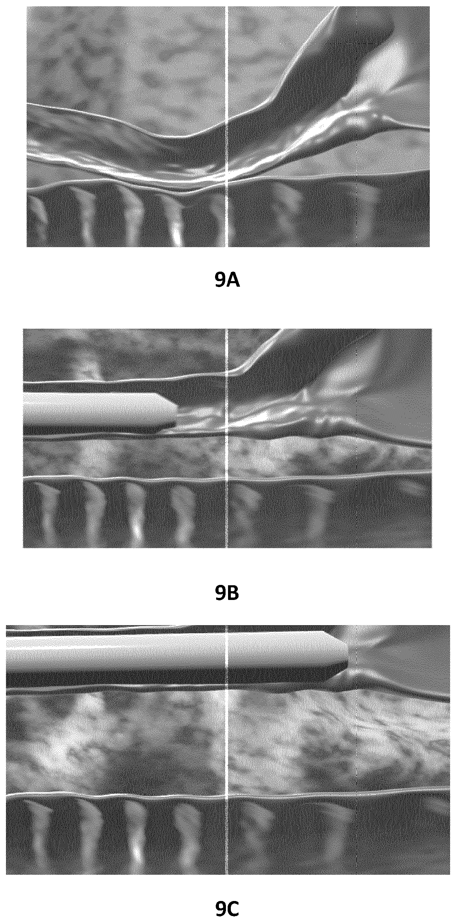

Next, the urethra is catheterized with the Introducer (2) by means of a mounted bladder tube, with the result of rectification and elevation of the same **(****figure 9B****).** In this way, when the urethra is raised, the sub urethral space will enlarge and allow access and placement of the mesh or network of threads (9) without risk of injury to the urethra **(****figure 9C****).**

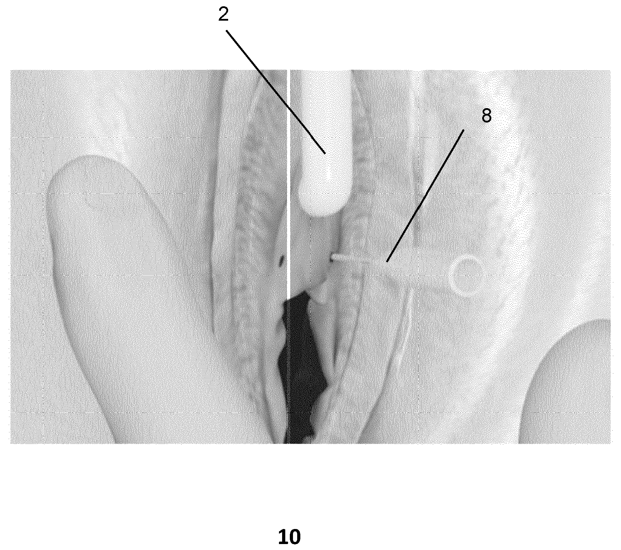

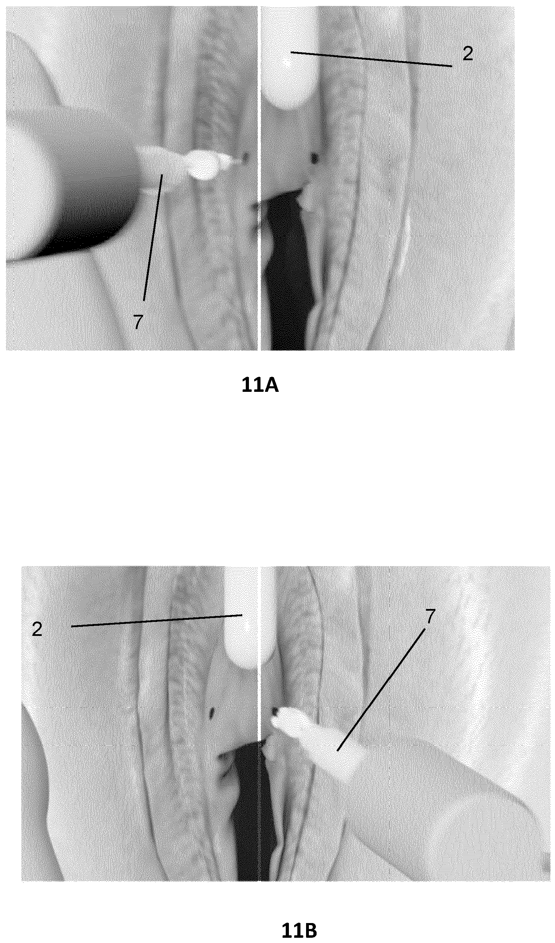

Once this is done, two entry holes are made at the base of the vestibule with a 19G needle (8), and after injection of 2 cc of 2% lidocaine with epinephrine **(****figure 10****),** a mesh thread (9) is mounted on the thread holder (7) and introduced through one of the holes. The inlet is perpendicular and at an angle of 75° to the end of the cannula **(****figures 11A and 11B****).** It is then removed, leaving the thread placed and crossed in the sub urethral space.

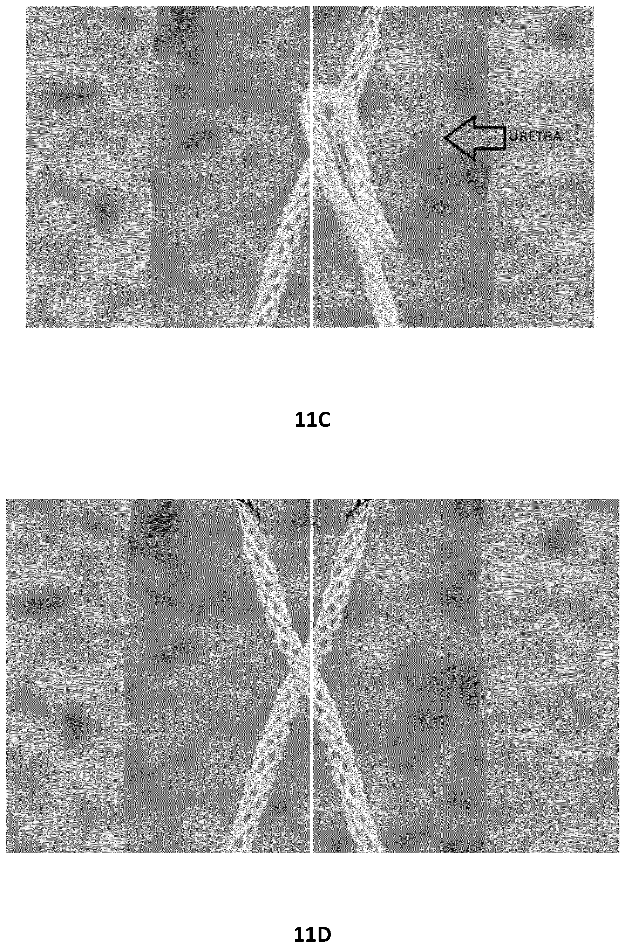

The same maneuver is repeated through the remaining hole, beginning the construction of the new support or floor of the urethra **(****figures 11C and 11D****).**

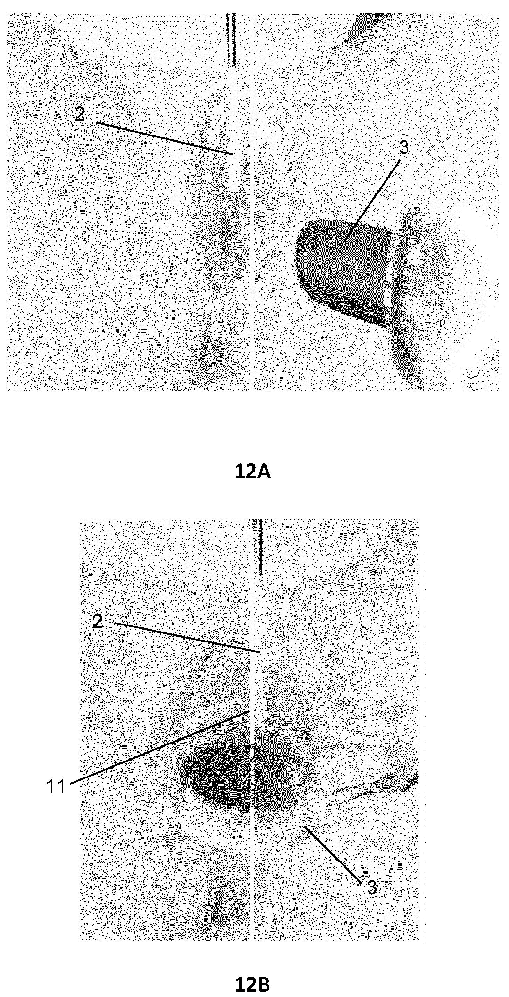

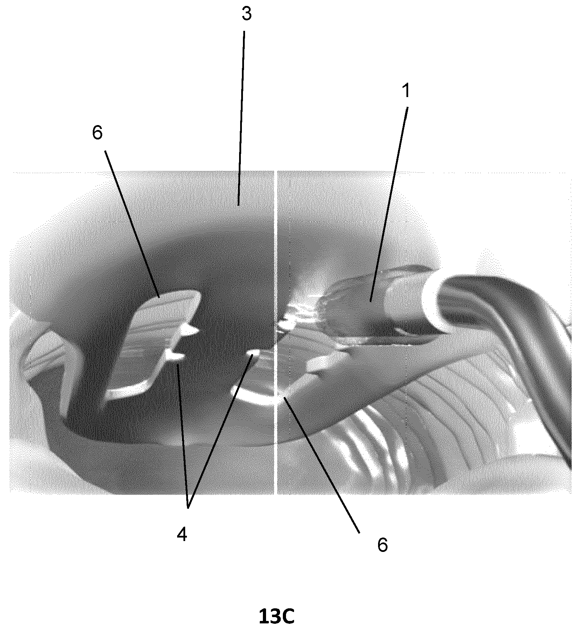

Then, the fenestrated speculum (3) is placed and rotated to 12 o'clock **(****figure 12 A),** fitting the introducer (2) in the notch (11) of the fenestrated speculum (3) and proceeding to open it **(****figure 12 B).** On the front face of the fenestrated speculum (3), and following the inclined grooves (4) of the two windows, an entrance hole is made with the needle (8) mounted on the thread holder (7) **(****figure 13 A),** and then the multi thread **(****figure 13 B)** mounted on the bayonet (1) is inserted, finally placing it in the sub urethral space and repeating the same maneuver in each of the inclined grooves (3) **(****figure 13 C).**

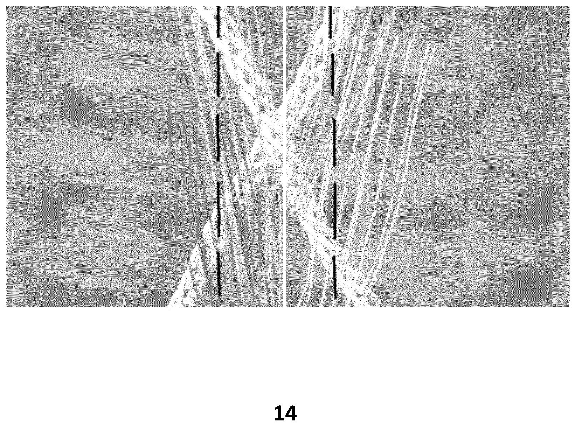

The figure 14 shows the final placing of the multi thread in the sub urethral space.

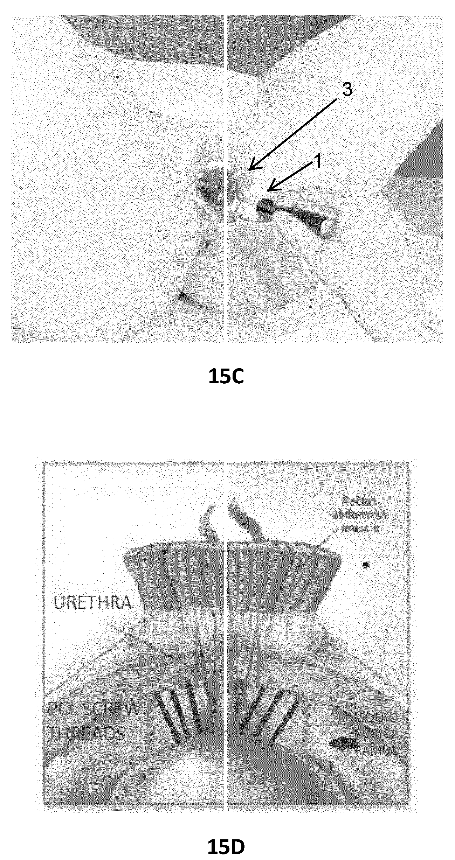

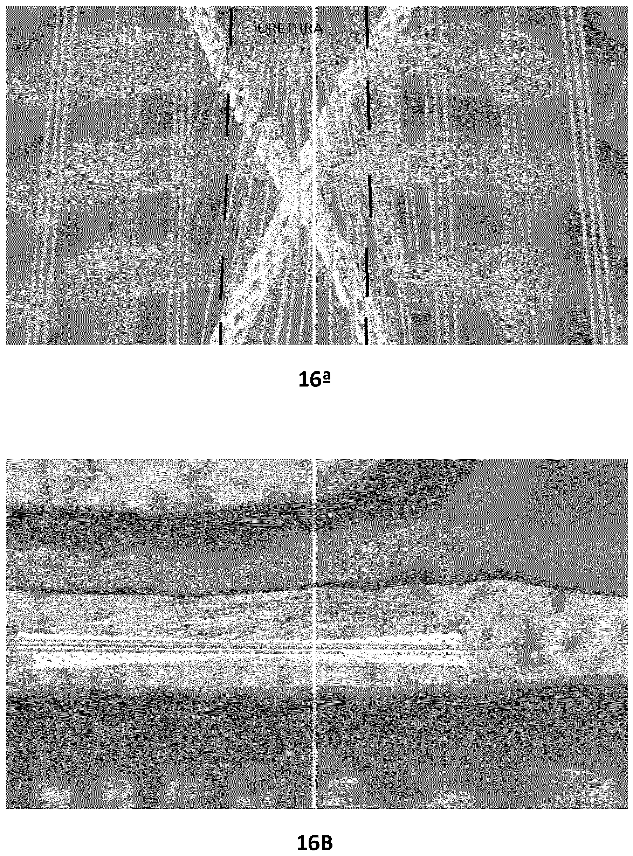

Finally, apply the monoscrew threads (10) mounted on needles (8) **(****figure 15B****)** with a cutting edge laterally to the urethra, entering the vaginal urethra space through the windows (6) of the fenestrated speculum (3) with the help of bayonet (1) **(****figures 15A** **and** **15C****).** The threads are placed by rotating the speculum three times to each side and placing three threads per movement **(****figure 15D****),** in this way - with the sub urethral network previously placed - the mesh is completed from one ischiopubic ramus to the other and from the urethral meatus to the bladder, providing a new support or floor to the urethra throughout its path.

## Claims

1. Instruments for supporting and placing absorbable threads in the suburethral space, for the non-surgical treatment of grade 3 stress urinary incontinence in women, comprising a fenestrated speculum (3), an introducer (2) element, a bayonet (1) and a thread holder (7),
**characterized in that** the fenestrated speculum (3) comprises:
- two lateral and longitudinal windows (6) 7 mm wide by 65 mm long,
- a central rib (5) between both windows (6),
- inclined grooves (4) on the central rib (5) for operator guidance at the inner edge of each window (6), intended to guide needles (8) with threads mounted on them, and
- a notch (11) which when the speculum (3) is in use is in the 12 o'clock position for fitting the introducer (2).

2. **The** instruments element of claim 1, wherein the introducer (2) comprises a handle followed by a straight portion at an angle of 70° between the handle and the straight portion, and a hook at its end, intended to enter the urethra through the urethral meatus and into the bladder.

3. **The** instruments of claim 1, wherein the bayonet (1) comprises a handle and a rod in the shape of an italic "S" and at an angle of 30°, which ends in a pointer intended to receive a cannula carrying a thread.

4. **The** instruments of claim 1, wherein the thread holder (7) comprises a cylindrical body and a pressure-adaptive pointer where the mesh threads are placed for placement.

## Patentansprüche

1. Instrumente zur Unterstützung und Platzierung resorbierbarer Fäden in der unterurethralen Kammer zur nicht-chirurgischen Behandlung von Stressinkontinenz der Stufe 3 bei Frauen, umfassend ein fenestriertes Spekulum (3), ein Einführelement (2), ein Bajonett (1) und einen Fadenhalter (7),
**dadurch gekennzeichnet, dass** das fenestrierte Spekulum (3) Folgendes umfasst:
- zwei seitliche und longitudinale Fenster (6) mit einer Breite von 7 mm und einer Länge von 65 mm,
- eine zentrale Rippe (5) zwischen beiden Fenstern (6),
- geneigte Nuten (4) an der zentralen Rippe (5) zur Führung des Anwenders am inneren Rand jedes Fensters (6), die dazu bestimmt sind, Nadeln (8) mit daran montierten Fäden zu führen, und
- eine Kerbe (11), die sich bei Verwendung des Spekulums (3) in der 12-Uhr-Position befindet, um die Einführhilfe (2) aufzunehmen.

2. Instrumentenelement nach Anspruch 1, wobei die Einführhilfe (2) einen Griff, gefolgt von einem geraden Abschnitt in einem Winkel von 70° zwischen dem Griff und dem geraden Abschnitt, und einen Haken an ihrem Ende umfasst, der dazu bestimmt ist, durch die Harnröhrenmündung in die Harnröhre und in die Blase einzudringen.

3. Instrumente nach Anspruch 1, wobei das Bajonett (1) einen Griff und einen Stab in Form eines schräg gestellten "S" und in einem Winkel von 30° umfasst, der in einer Spitze endet, die dazu bestimmt ist, eine einen Faden tragende Kanüle aufzunehmen.

4. Instrumente nach Anspruch 1, wobei der Fadenhalter (7) einen zylindrischen Körper und eine druckadaptive Spitze umfasst, an der die Netzfäden zum Platzieren platziert sind.

## Revendications

1. Instruments pour le support et la mise en place de fils résorbables dans l'espace sous-urétral, pour le traitement non chirurgical de l'incontinence urinaire d'effort de grade 3 chez la femme, comprenant un spéculum fenêtré (3), un élément introducteur (2), une baïonnette (1) et un porte-fil (7),
**caractérisés en ce que** le spéculum fenêtré (3) comprend :
- deux fenêtres latérales et longitudinales (6) de 7 mm de large sur 65 mm de long,
- une nervure centrale (5) entre les deux fenêtres (6),
- des rainures inclinées (4) sur la nervure centrale (5) pour le guidage de l'opérateur au niveau du bord interne de chaque fenêtre (6), destinées à guider des aiguilles (8) sur lesquelles sont montés des fils, et
- une encoche (11) qui, lorsque le spéculum (3) est en cours d'utilisation, se trouve à la position 12 heures pour l'ajustement de l'introducteur (2).

2. Élément d'instruments selon la revendication 1, dans lequel l'introducteur (2) comprend un manche suivi d'une partie rectiligne formant un angle de 70° entre la poignée et la partie droite, et un crochet au niveau de son extrémité, destiné à pénétrer dans l'urètre par le méat urétral et dans la vessie.

3. Instruments selon la revendication 1, dans lesquels la baïonnette (1) comprend un manche et une tige sous la forme d'un « S » italique et formant un angle de 30°, qui se termine par un pointeur destiné à recevoir une canule portant un fil.

4. Instruments selon la revendication 1, dans lesquels le porte-fil (7) comprend un corps cylindrique et un pointeur adaptable à la pression où les fils de maille sont placés pour leur mise en place.
